# EUROPÄISCHE PATENTANMELDUNG

(11) **EP 4 109 093 A1**
(43) Veröffentlichungstag der Anmeldung: **28.12.2022**
(21) Anmeldenummer: 22179816.8
(22) Anmeldetag: 20.06.2022
(51) Int. Cl.: G01N 33/28

(54) **VERFAHREN ZUR ERMITTLUNG DES ZUSTANDES VON TECHNISCHEN SYSTEMEN**

(30) Priorität: 23.06.2021 DE 102021116192
(71) Anmelder: Lufthansa Technik AG, 22335 Hamburg (DE)
(72) Erfinder: Seibold, Björn, 64625 Bensheim (DE); Deja, Dirk, 64569 Nauheim (DE)
(74) Vertreter: Glawe, Delfs, Moll

(57) **Zusammenfassung**

Die Erfindung betrifft ein Verfahren zur Ermittlung des Zustandes von technischen Systemen, insbesondere von solchen in Flugzeugen, durch Analyse von Betriebsflüssigkeiten, mit den Schritten:
a) Entnahme einer Probe der zu analysierenden Betriebsflüssigkeit;
b) Bestimmen von Größen betreffend die Anzahl und/oder Größenverteilung von Partikeln in der Probe der Betriebsflüssigkeit mithilfe von Einzelpartikel-Massenspektrometrie mit induktiv gekoppeltem Plasma;
c) Ermitteln wenigstens eines charakteristischen Parameters aus den bestimmten Größen durch multivariate Datenanalyse;
d) Wiederholen der vorhergehenden Schritte und Beobachten der Entwicklung des wenigstens einen ermittelten charakteristischen Parameter; und
e) Evaluieren des wenigstens einen ermittelten Parameter und dessen Entwicklung zur Vorhersage des künftigen Zustandes des technischen Systems und/oder erforderlicher Wartungsarbeiten.

## Beschreibung

Die Erfindung betrifft ein Verfahren zur Ermittlung des Zustandes von technischen Systemen, insbesondere von solchen in Flugzeugen, durch Analyse von Betriebsflüssigkeiten.

Im Stand der Technik ist grundsätzlich bekannt, dass sich der Zustand eines technischen Systems indirekt durch Untersuchung von darin verwendeten Betriebsmitteln ermitteln oder zumindest abschätzen lässt. So lässt sich im einfachsten Fall durch die Feststellung von Metallpartikeln im Motoröl eine mechanische Abnutzung einer mit dem Motoröl unmittelbar in Kontakt kommenden Motorkomponente erkennen.

Es ist bekannt, den Zustand von technischen Systemen durch die Ermittlung einzelner absoluter Messgrößen von Betriebsflüssigkeiten, wie bspw. der Konzentration bestimmter Stoffe und Materialien in der Betriebsflüssigkeit oder der Viskosität der Betriebsflüssigkeit, abzuleiten. Dazu muss eine Probe der Betriebsflüssigkeit mit Hilfe festgelegter und insbesondere normierter Verfahren geprüft werden. Ein Beispiel hierfür ist das "Spetrometric Oil Analysis Program" (SOAP), bei dem Öl aus Flugzeugtriebwerken und Gasturbinen spektrometrisch untersucht wird, um so die Gesamtkonzentrationen der im Medium - also im Öl - detektierten Elemente zu ermitteln. Allerdings können mit diesem Verfahren typischerweise keine einzelnen Partikel charakterisiert werden und die Vorhersagen beruhen allein auf Basis der Gesamtkonzentration einzelner Elemente, wobei in der Regel lediglich Partikel mit Größen kleiner ca. 5 µm vollständig erfasst werden können. Auch ermöglicht das SOAP-Verfahren keine Unterscheidung zwischen im Fluid gelöster und als Partikel vorliegender Elemente. Im Ergebnis ist die Aussagekraft dieses Verfahrens begrenzt, denn es leidet darunter, dass Vorhersagen allein auf Basis der Gesamtkonzentration einzelner Elemente in dem Medium häufig zu unsicher sind, um daraus mit gebotener Genauigkeit den Zustand des technischen Systems abzuleiten. In der Folge kann durch das SOAP-Verfahren eine Fehlfunktion des technischen Systems nur wenige Betriebsstunden vor dem tatsächlichen Ausfall angezeigt werden.

Aus den Patentveröffentlichungen US 3,981,584 oder US 2014/0121994 A1 sind weitergehende Verfahren bekannt, bei denen die Partikel aus der Betriebsflüssigkeit herausgefiltert werden, um sie anschließend spektrometrisch (OES) bzw. mit einem Rasterelektronenmikroskop (SEM) in Kombination mit Röntgenfluoreszenzanalyse (RFA) nach chemischer Zusammensetzung, Größe und Morphologie für eine genauere Vorhersage zu klassifizieren. Diese Verfahren sind in der Vorbereitung und Durchführung jedoch wesentlich aufwendiger und daher für die Anwendung im täglichen Betrieb ungeeignet.

Alternativ sind sog. Online-Systeme bekannt, die über den Zustand des Systems Auskunft gebende Messgrößen während des Betriebs des technischen Systems erfassen. Ein Beispiel hierfür ist die Messung eines elektromagnetischen Feldes über eine mit der Betriebsflüssigkeit gefüllten Messstrecke, bspw. über den Querschnitt einer Förderleitung für die Betriebsflüssigkeit. Veränderungen im elektrischen Feld lassen Schlüsse auf in der Betriebsflüssigkeit mitgeführte Feststoffe, wie bspw. Metallspäne in einem Betriebsöl (vgl. Oil Debris Monitor; ODM), zu. Durch entsprechende Detektoren können zwar bestimmte, ggf. problematische (Fehler-)Zustände von technischen Systemen erkannt werden, aufgrund der fehlenden Selektivität gegenüber der Gesamtheit der im Öl vorhandenen Analyten, lassen diese Systeme aber keine tiefergehenden und detaillierten Rückschlüsse auf den Zustand des technischen Systems zu.

Aufgabe der vorliegenden Erfindung ist es, ein Verfahren zur Ermittlung des Zustandes von technischen Systemen zu schaffen, welches die aus dem Stand der Technik bekannten Nachteile nicht mehr oder nur noch in vermindertem Umfang aufweist.

Gelöst wird diese Aufgabe durch ein Verfahren gemäß dem Hauptanspruch. Vorteilhafte Weiterbildungen sind Gegenstand der abhängigen Ansprüche.

Demnach betrifft die Erfindung ein Verfahren zur Ermittlung des Zustandes von technischen Systemen durch Analyse von Betriebsflüssigkeiten, mit den Schritten:
a) Entnahme einer Probe der zu analysierenden Betriebsflüssigkeit;
b) Bestimmen von Größen betreffend die Anzahl und/oder Größenverteilung von Partikeln in der Betriebsflüssigkeit mithilfe von Einzelpartikel-Massenspektrometrie mit induktiv gekoppeltem Plasma;
c) Ermitteln wenigstens eines charakteristischen Parameters aus den bestimmten Größen durch multivariate Datenanalyse;
d) Wiederholen der vorhergehenden Schritte und Beobachten der Entwicklung des wenigstens einen ermittelten charakteristischen Parameter; und
e) Evaluieren des wenigstens einen ermittelten Parameter und dessen Entwicklung zur Vorhersage des künftigen Zustandes des technischen Systems und/oder erforderlicher Wartungsarbeiten.

Die Erfindung hat erkannt, dass durch den kombinierten Einsatz von Einzelpartikel-Massenspektrometrie über induktiv gekoppeltes Plasma sowie multivariate Datenanalyse eine detaillierte Ermittlung bzw. Vorhersage des Zustandes eines technischen Systems möglich ist. Dabei ist das erfindungsgemäße Verfahren gegenüber den aus dem Stand der Technik bekannten Verfahren effizienter, da einerseits nur überschaubarer Aufwand für die Gewinnung von Proben der zu analysierenden Betriebsflüssigkeiten und der Durchführung der übrigen Verfahrensschritte erforderlich ist, andererseits liefert das erfindungsgemäße Verfahren deutlich detailliertere und somit insbesondere aussagekräftigere Ergebnisse als die meisten bekannten Verfahren.

In einem ersten Schritt des erfindungsgemäßen Verfahrens wird eine Probe der zu untersuchenden Betriebsflüssigkeit entnommen. Dabei werden keine besonderen Anforderungen an die Probenentnahme gestellt, sodass die Probenentnahme auch von Personen durchgeführt werden kann, die nicht besonders dafür geschult sein müssen.

In einem zweiten Schritt werden mithilfe von Einzelpartikel-Massenspektrometrie mit induktiv gekoppeltem Plasma diverse Größen für die zuvor entnommene Probe der Betriebsflüssigkeit ermittelt. Diese an sich bekannte Analysemethode erlaubt die teilweise Charakterisierung der in der Betriebsflüssigkeit enthaltenen Partikels sowie deren jeweilige Anzahl und/ Größenverteilung. Die Massenspektrometrie mit induktiv gekoppeltem Plasma erlaubt die Detektion von Elementen mit sehr hoher Empfindlichkeit und kann daher Nanopartikel bis zu einer Größe von kleiner als 10 nm für viele Elemente nachweisen. Geeignete Geräte zur Durchführung der Einzelpartikel-Massenspektrometrie mit induktiv gekoppeltem Plasma sind bspw. PlasmaQuant^{®} MS" der Firma AnalytikJena AG, Jena, Deutschland, oder "350 ICP-MS" der Firma Perkin Elmer Inc., Waltham, MA, USA.

Es ist bevorzugt, wenn zur Bestimmung von Anzahl und/oder Größenverteilung von Partikeln in der Betriebsflüssigkeit eine repräsentative Anzahl von Einzelmessungen durchgeführt wird, deren Ergebnisse, vorzugsweise nach Entfernung von möglichen Rauschen und/oder Normierung, in eine standardisierte Darstellung der Ergebnisse in Form von Größen mit einer oder mehreren Verteilungsfunktionen überführt wird. Indem mehrere Einzelmessungen durchgeführt werden, die anschließend mithilfe einer oder mehrerer Verteilungsfunktionen in eine standardisierte Darstellung überführt werden, können unerwünschte Beeinträchtigungen durch Messungenauigkeiten reduziert werden. Gleiches gilt für die optionale Entfernung von möglichem Rauschen und/oder die Normierung der Messergebnisse. Auch kann der Speicherbedarf für die Messergebnisse reduziert werden.

Aus den durch die Einzelpartikel-Massenspektrometrie mit induktiv gekoppeltem Plasma ermittelten Größen wird anschließend mithilfe von multivariater Datenanalyse wenigstens ein charakteristischer Parameter, in der Regel eine Vielzahl entsprechender Parameter, ermittelt. Bei einer entsprechenden multivariater Datenanalyse wird die Zusammenhangsstruktur der Daten analysiert, um den Anzahl der ermittelten Größen in eine geringere Anzahl charakteristischer Parameter zu überführen, ohne die darin enthaltenen Informationen wesentlich zu reduzieren. Entsprechende Datenanalysen sind im Stand der Technik bekannt.

Dabei ist bevorzugt, wenn die multivariate Datenanalyse unabhängig von absoluten und/oder kalibrierten Größen erfolgt. Indem die multivariate Datenanalyse in der Folge nur auf die Relationen zwischen den einzelnen ermittelten Größen abstellt, kann auf eine genaue Kalibrierung des Geräts zur Einzelpartikel-Massenspektrometrie mit induktiv gekoppeltem Plasma, bspw. mithilfe von geeichten Proben zur Ermittlung von Absolutwerten o.ä., verzichtet werden. Durch den Verzicht auf eine Kalibrierung, bspw. zur Ermittlung von absoluten Größen, ist weiterhin auch die potenzielle Fehlerquelle durch fehlkalibrierte Geräte ausgeschlossen.

Bereits aus den so ermittelten charakteristischen Parameter lassen sich ggf. Informationen über den Zustand eines technischen Systems ableiten. Es hat sich aber gezeigt, dass sich der Zustand des technischen Systems, von dem die analysierte Betriebsflüssigkeit stammt, besonders genau ermitteln und sogar vorhersagen lässt, wenn die beschriebenen Schritte von der Entnahme der Probe der zu analysierenden Betriebsflüssigkeit bis zur Ermittlung wenigstens eines charakteristischen Parameters in einigem zeitlichen Abstand wiederholt werden und dann die Entwicklung der einzelnen ermittelten charakteristischen Parameter beobachtet wird.

Die Wiederholung der genannten Schritte erfolgt dabei in regelmäßigen Abständen, womit die Beobachtung der Veränderung der charakteristischen Parameter vereinfacht wird. Für die meisten technischen Systeme ist eine regelmäßige Wiederholung in Abständen von einer oder zwei Wochen ausreichend.

Um zu verhindern, dass fehlerhaft bestimmte Größen und/oder falsch ermittelte charakteristische Parameter berücksichtigt werden, ist bevorzugt, wenn das Beobachten der Entwicklung des wenigstens einen ermittelten Parameters eine Prüfung der statistischen Signifikanz des zuletzt ermittelten wenigstens einen Parameters und/oder eine Regressionsanalyse mittels Kalibrierfunktion umfasst. Durch entsprechende Prüfungen können technisch unsinnige Parameter erkannt und von der Beobachtung ausgeschlossen werden.

Die ermittelten charakteristischen Parameter und die darauf basierenden beobachteten Entwicklungen werden abschließend evaluiert, um den künftigen Zustand des technischen Systems und/oder erforderliche Wartungsarbeiten vorhersagen zu können. Durch geeignete Evaluation kann festgestellt werden, ob oder wann ein technisches System gewartet werden muss, bspw. weil Änderungen in der Betriebsflüssigkeit auf einen Verschleiß bestimmter Komponenten hindeuten. Das Evaluieren des wenigstens einen ermittelten Parameter und dessen Entwicklung kann dabei auch den Abgleich mit Erwartungsmustern umfassen. Spiegelt das Erwartungsmuster aufgrund von Verschleiß zu erwartende Änderungen wieder, kann eine Abweichung vom Erwartungsmuster auf eine unvorhersehbare Fehlfunktion des technischen Systems hindeuten.

Als zu analysierende Betriebsflüssigkeit kommen grundsätzlich Motoröl oder andere Schmiermittel, Hydrauliköl, Kühlmittel oder Kerosin in Betracht. Bei dem technischen System, dessen Zustand ermittelt werden soll, handelt es sich vorzugsweise um ein Flugzeugsystem, d. h. ein technisches System eines Flugzeuges, wie bspw. ein Triebwerk oder ein Hydrauliksystem.

Neben der Anzahl und/oder Größenverteilung von Partikeln in der Probe der Betriebsflüssigkeit können noch weitere chemische und/oder physikalische Eigenschaften der entnommenen Betriebsflüssigkeit, wie bspw. die Säurezahl oder die Viskosität, bei der beschriebenen Analyse berücksichtigt werden. Die entsprechenden Eigenschaften können mit geeigneten bekannten Verfahren ermittelt und bereits bei der multivariaten Datenanalyse, spätestens aber beim Evaluieren berücksichtigt werden.

Es ist anzumerken, dass Teile des erfindungsgemäßen Verfahrens in anderen Bereichen der Technik, wie bspw. der Pharmaindustrie, bereits bekannt sein mögen. Allerdings sind die Anwendung von Einzelpartikel-Massenspektrometrie über induktiv gekoppeltes Plasma und/oder die multivariate Datenanalyse zur Ermittlung des Zustandes von technischen Systemen im Stand der Technik gänzlich unbekannt.

Die Erfindung wird nun anhand einer vorteilhaften Ausführungsform unter Bezugnahme auf die beigefügten Zeichnungen beispielhaft beschrieben. Es zeigen:

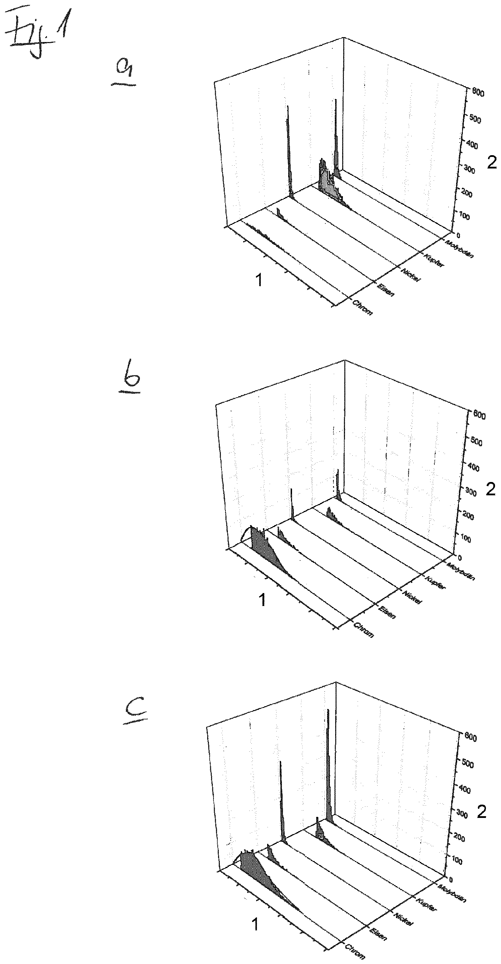

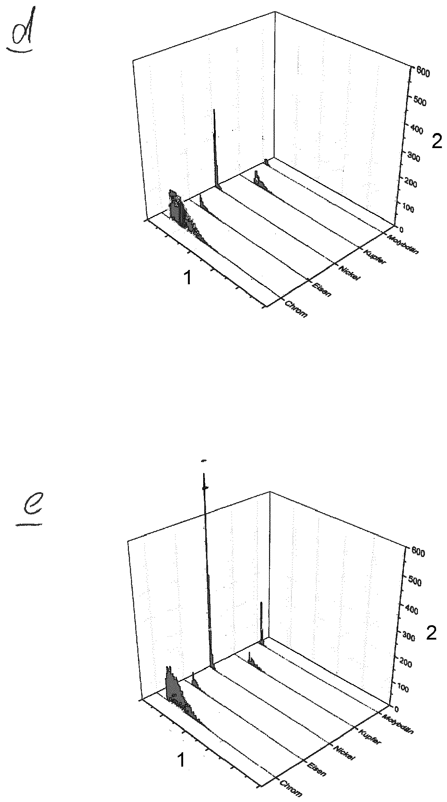

- Figur 1a-e:: Ergebnisse von in zeitlichen Abständen durchgeführten Untersuchungen durch Einzelpartikel-Massenspektrometrie mit induktiv gekoppeltem Plasma von Öl eines Flugzeugtriebwerks.

Bei der nachfolgend beschriebenen Durchführung des erfindungsgemäßen Verfahrens zur Ermittlung des Zustandes eines technischen Systems wurde in Abständen von 2 Wochen eine Ölprobe aus einem Flugzeugtriebwerk entnommen, wobei die Ergebnisse in Figuren 1a-d chronologisch angeordnet sind. Bei dem technischen System handelt es sich somit um ein Flugzeugtriebwerk, dessen Öl eine Betriebsflüssigkeit im Sinne der Erfindung darstellt.

Von den entnommenen Proben der zu analysierenden Betriebsflüssigkeit werden Größen betreffend die Anzahl und die Größenverteilung von Partikeln mithilfe von Einzelpartikel-Massenspektrometrie mit induktiv gekoppeltem Plasma ermittelt. Im hier beschrieben Ausführungsbeispiel wurde dazu das Gerät "PlasmaQuant^{®} MS" der Firma AnalytikJena AG, Jena, Deutschland, verwendet. Das Gerät wurde dabei für keine der durchgeführten Messungen in irgendeiner Form kalibriert, sodass die ermittelten Größen nicht als Absolutwerte angesehen werden können. Allerdings wurden die Einstellungen des Gerätes über den gesamten Untersuchungszeitraum nicht verändert, sodass die Ergebnisse zu den unterschiedlichen Zeitpunkten durchaus miteinander vergleichbar sind.

Durch die Einzelpartikel-Massenspektrometrie mit induktiv gekoppeltem Plasma, die für jede Probe mehrfach durchgeführt wird, wobei die Einzelergebnisse dann gemittelt werden, werden die Zählimpulse 1 und die Größenverteilung 2 von Partikeln bestimmter Stoffe im zu analysierenden Betriebsstoff ermittelt. Im dargestellten Ausführungsbeispiel können die Partikel aus Chrom, Eisen, Nickel, Kupfer oder Molybdän sein.

Für zumindest einen Teil der so ermittelten Partikelgrößenverteilungen für einzelne Stoffe können Verteilungsfunktionen angepasst werden, die eine vereinfachte standardisierte Darstellung ermöglichen. So ist beispielhaft in Figuren 1b, c jeweils eine Verteilungsfunktion für Chrom dargestellt.

Wie bereits dargelegt, ist das verwendete Gerät nicht besonders kalibriert, sodass die ermittelten Größen nicht als Absolutwerte angesehen werden können. Dennoch lassen sich über multivariate Datenanalyse charakteristische Parameter ermitteln, anhand derer der Zustand des technischen Systems abgelesen werden kann.

Im dargestellten Ausführungsbeispiel soll vereinfacht das Verhältnis der Maxima der Partikelgrößenverteilung 2 von Nickel und Molybdän und der Mittelwert der Zählimpulse 1 für Nickel und Molybdän als ein beispielhafter charakteristischer Parameter herangezogen werden. Für die in Figuren 1a-e dargestellten Probenzeitpunkte ergibt sich :

| **Zeitpunkt** | **Verhältnis Max(Ni)/Max(Mo)** | **Mittelwert Count (Ni), Count(Mo)** |
|---|---|---|
| T=1 (Figur 1a) | ≈ 1,14 | 375 |
| T=2 (Figur 1b) | ≈ 1,07 | 145 |
| T=3 (Figur 1c) | ≈ 0,72 | 430 |
| T=4 (Figur 1d) | ≈ 10,33 | 170 |
| T=5 (Figur 1e) | ≈ 4,57 | 585 |

Die zu den einzelnen Zeitpunkten ermittelten Parameter können verschiedentlich evaluiert werden.

Im vorliegenden Ausführungsbeispiel ist als erstes Kriterium festgelegt, dass er Mittelwert der Zählimpulse 1 für Nickel und Molybdän über 400 liegen soll. Als zweites Kriterium soll das Verhältnis Maxima der Partikelgrößenverteilung 2 von Nickel und Molybdän zwischen 0,7 und 1,3 sein.

Eine Warnung wird nur dann ausgegeben, wenn beide Kriterien erfüllt sind. In den in den Figuren dargestellten und in der Tabelle zusammengefassten Beispielen wird somit nur zu dem Zeitpunkt T=3 (Figur 1c) eine Warnung ausgegeben.

Alternativ kann ein Erwartungsmuster für den fraglichen Parameter des Verhältnisses der Maxima der Partikelgrößenverteilung 2 vorliegen, aus dem sich ergibt, dass nach einem Absinken des Parameters auf einen Wert unterhalb 1,0 (hier zum Zeitpunkt T=3) kurzfristig ein Anstieg auf einen Wert von über 3,0 zu erwarten ist (hier zum Zeitpunkt T=4), der aber dann keinen unmittelbaren Wartungseingriff erfordert, wenn der Wert anschließend wieder absinkt (vgl. Zeitpunkt T=5). Erst wenn der Wert dauerhaft - d. h. kontinuierlich über eine vorgegebene Anzahl an Analysezeitpunkten - oberhalb von 3,0 verbleibt, wird eine geeignete Warnung ausgegeben bzw. eine geeignete Wartung vorgesehen.

## Patentansprüche

1. Verfahren zur Ermittlung des Zustandes von technischen Systemen durch Analyse von Betriebsflüssigkeiten, mit den Schritten:
a) Entnahme einer Probe der zu analysierenden Betriebsflüssigkeit;
b) Bestimmen von Größen betreffend die Anzahl und/oder Größenverteilung von Partikeln in der Probe der Betriebsflüssigkeit mithilfe von Einzelpartikel-Massenspektrometrie mit induktiv gekoppeltem Plasma;
c) Ermitteln wenigstens eines charakteristischen Parameters aus den bestimmten Größen durch multivariate Datenanalyse;
d) Wiederholen der vorhergehenden Schritte und Beobachten der Entwicklung des wenigstens einen ermittelten charakteristischen Parameter; und
e) Evaluieren des wenigstens einen ermittelten Parameter und dessen Entwicklung zur Vorhersage des künftigen Zustandes des technischen Systems und/oder erforderlicher Wartungsarbeiten.

2. Verfahren nach Anspruch 1,
**dadurch gekennzeichnet, dass**
zur Bestimmung von Anzahl und/oder Größenverteilung von Partikeln in der Betriebsflüssigkeit eine repräsentative Anzahl von Einzelmessungen durchgeführt wird, deren Ergebnisse, vorzugsweise nach Entfernung von möglichen Rauschen und/oder Normierung, in eine standardisierte Darstellung der Ergebnisse mit einer oder mehreren Verteilungsfunktionen überführt wird.

3. Verfahren nach Anspruch 1 oder 2,
**dadurch gekennzeichnet, dass**
die multivariate Datenanalyse unabhängig von absoluten und/oder kalibrierten Größen erfolgt.

4. Verfahren nach einem der vorhergehenden Ansprüche,
**dadurch gekennzeichnet, dass**
das Wiederholen der Schritte (a) bis (c) in regelmäßigen Abständen, vorzugsweise in Abständen von einer oder zwei Wochen, durchgeführt wird.

5. Verfahren nach einem der vorhergehenden Ansprüche,
**dadurch gekennzeichnet, dass**
das Beobachten der Entwicklung des wenigstens einen ermittelten Parameters eine Prüfung der statistischen Signifikanz des zuletzt ermittelten wenigstens einen Parameters und/oder eine Regressionsanalyse mittels Kalibrierfunktion umfasst.

6. Verfahren nach einem der vorhergehenden Ansprüche,
**dadurch gekennzeichnet, dass**
Evaluieren des wenigstens einen ermittelten Parameter und dessen Entwicklung den Abgleich mit Erwartungsmustern umfasst.

7. Verfahren nach einem der vorhergehenden Ansprüche,
**dadurch gekennzeichnet, dass**
neben der Zählimpulse (1) und/oder Größenverteilung (2) von Partikeln in der Probe der Betriebsflüssigkeit noch weitere chemische und/oder physikalische Eigenschaften der entnommenen Betriebsflüssigkeit berücksichtigt werden.

8. Verfahren nach einem der vorhergehenden Ansprüche,
**dadurch gekennzeichnet, dass**
die zu analysierende Betriebsflüssigkeit Motoröl oder anderes Schmiermittel, Hydrauliköl, Kühlmittel oder Kerosin ist.

9. Verfahren nach einem der vorhergehenden Ansprüche,
**dadurch gekennzeichnet, dass**
das technische System, dessen Zustand ermittelt werden soll, ein Flugzeugsystem ist.
